(19) Europäisches Patentamt
European Patent Office
Office européen des brevets

(11) Veröffentlichungsnummer: **0 274 124**
**A1**

# (12) EUROPÄISCHE PATENTANMELDUNG

(21) Anmeldenummer: **87119218.3**

(22) Anmeldetag: **24.12.87**

(51) Int. Cl.⁴: **C07D 211/78**, C07D 409/04, A61K 31/44

(30) Priorität: **08.01.87 DE 3700307**

(43) Veröffentlichungstag der Anmeldung:
**13.07.88 Patentblatt 88/28**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Meyer, Horst, Dr.**
**Miles Inc. 400 Morgan Lane**
**West Haven, CT 06516(US)**
Erfinder: **Schwenner, Eckhard, Dr.**
**Paul-Ehrlich Strasse 29**
**D-5600 Wuppertal 1(DE)**
Erfinder: **Knorr, Andreas, Dr.**
**Trillser Graben 10**
**D-4006 Erkrath 2(DE)**

## (54) Tetrahydropyridine, Verfahren zu ihrer Herstellung und ihre Verwendung in Arzneimitteln.

(57) Die vorliegende Erfindung betrifft neue Tetrahydropyridinverbindungen der allgemeinen Formel I

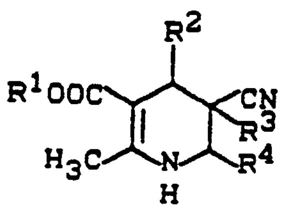

(I)

in welcher $R^1$, $R^2$, $R^3$ und $R^4$ die in der Beschriebung angegebene Bedeutung haben, ein Verfahren zu ihrer Herstellung sowie ihre Verwendung in Arzneimitteln, insbesondere in kreislaufbeeinflussenden Arzneimitteln.

EP 0 274 124 A1

# Tetrahydropyridine, Verfahren zu ihrer Herstellung und ihre Verwendung in Arzneimitteln

Die vorliegende Erfindung betrifft neue Tetrahydropyridinverbindungen, ein Verfahren zu ihrer Herstellung sowie ihre Verwendung in Arzneimitteln, insbesonderes in kreislaufbeeinflussenden Arzneimitteln.

Die Erfindung betrifft Tetrahydropyridinverbindungen der allgemeinen Formel (I)

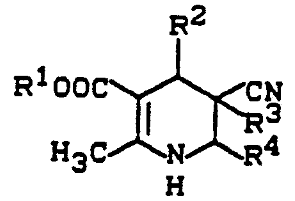

(I)

in welcher

$R^1$ -für einen geradkettigen, verzweigten oder cyclischen, gesättigten oder ungesättigten Kohlenwasserstoffrest mit bis zu 20 Kohlenstoffatomen steht, der durch ein Sauerstoffatom oder ein Schwefelatom in der Kette unterbrochen sein kann und/oder der substituiert sein kann durch Halogen, Cyano, Hydroxy, Acyloxy, Nitro, Nitrooxy oder durch eine gegebenenfalls durch Halogen, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substituierte Phenyl-, Phenoxy-, Phenylthio-oder Phenylsulfonylgruppe, oder durch eine α-, β-, oder γ-Pyridylgruppe oder durch eine Aminogruppe, wobei diese Aminogruppe, 2 gleiche oder verschiedene Substituenten aus der Gruppe Alkyl mit bis zu 4 Kohlenstoffatomen, Alkoxyalkyl mit bis zu 4 Kohlenstoffatomen, Phenyl oder Aralkyl trägt oder wobei diese Substituenten gegebenenfalls mit dem Stickstoffatom einen fünf-bis seibengliedrigen Ring bilden, der als weiteres heteroatom ein Sauerstoff-oder Schwefelatom, oder eine N-Phenyl-oder N-Alkylgruppierung enthalten kann, wobei diese Alkylgruppe 1 bis 3 Kohlenstoffatome umfaßt,

$R^2$ -für Phenyl, Naphtyl, Thienyl, Furyl, Pyrryl, Pyrazolyl, Imidazoyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazoyl, Benzoxadiazolyl, Benzothiadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Trimethylen, Tetramethylen, Pentamethylen, Dioxymethylen, Dioxyethylen, Halogen, Trifluormethyl, Trifluormethoxy, Difluormethoxy, Tetrafluorethoxy, Nitro, Cyano oder $SO_m$-Alkyl, worin m eine Zahl von 0 bis 2 bedeutet und Alkyl vorzugsweise 1 bis 4 Kohlenstoffatome mit gegebenenfalls 1 bis 3 Fluoratomen enthält, substituiert sein können,

$R^3$ -für eine Gruppe der Formel -CN oder $-COOR^5$ steht,
worin
$R^5$ -die gleiche Bedeutung hat wie $R^1$ und mit diesem gleich oder verschieden sein kann
und

$R^4$ -für Wasserstoff steht, oder
-für Phenyl oder Naphthyl steht, wobei die genannten Ringsysteme durch bis zu 2 gleiche oder verschiedene Substituenten der Reihe Nitro, Cyano, Alkyl mit 1 bis 4 Kohlenstoffatomen, Alkoxy mit 1 bis 4 Kohlenstoffatomen, Halogen, Trifluormethyl, Trifluormethoxy oder Trifluormethylthio substituiert sein können,
sowie ihre pharmazeutisch unbedenklichen Säureadditionssalze.

Die erfindungsgemäßen Verbindungen existieren in stereoisomeren Formen, die sich entweder wie Bild und Speigelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diasteromeren in bekannter Weise in die stereoisomer einheitlichen Bestandteile trennen (vg. E.L. Eliel, Stereochemistry of Carbon Compounds, McGraw Hill, 1962).

Pharmazeutisch unbedenklische Salze können Salze der erfindungsgemäßen Verbindungen mit

anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze der erfindungsgemäßen Verbindungen mit organischen Carbonsäuren oder Sulfonsäuren wie beispielsweise Ameisensäure, Essigsäure, Propionsäure, Maleinsäure, Fumarsäure, Äpfelsäure, Zitronensäure, Weinsäure, Milchsäure, Malonsäure, Benzoesäure, oder Methansulfonsäure, Ethansulfonsäure, Phenylsulfonsäure, Touolsulfonsäure oder Naphthalindisulfonsäure.

Bevorzugt seien Verbindungen der allgemeinen Formel (I) genannt, in welcher

$R^1$ -für einen geradkettigen, verzweigten oder cyclischen, gesättigten oder ungesättigen Kohlenwasserstoffrest mit bis zu 14 Kohlenstoffatomen steht, der durch ein Sauerstoffatom in der Kette unterbrochen sein kann und/oder der substituiert sein kann durch Fluor, Chlor, Brom, Iod, Cyano, Hyroxy, Acetoxy, Nitrooxy oder durch eine gegebenenfalls durch Fluor, Chlor, Brom, Alkoxy mit 1 bis 4 Kohlenstoffatomen, Alkyl mit bis 1 bis 4 Kohlenstoffatomen oder Trifluormethyl substituierte Phenyl - oder Phenoxygruppe, oder durch eine α-, β-oder γ-Pyridylgruppe oder durch eine Aminogruppe, wobei diese Aminogruppe 2 gleiche oder verschiedene Substituenten aus der Gruppe Alkyl mit 1 bis 4 Kohlenstoffatomen, Phenyl oder Benzyl trägt,

$R^2$ -für Phenyl, Naphthyl, Thienyl, Furyl, Pyridyl, Pyrimidyl, Chinolyl oder Benzoxadiazolyl steht, wobei die genannten Ringsysteme durch 1 bis 2 gleiche oder verschiedene Substituenten aus der Gruppe Phenyl, Alkyl mit 1 bis 4 Kohlenstoffatomen, Alkoxy mit 1 bis 2 Kohlenstoffatomen, Dioxymethylen, Fluor, Chlor, Brom, Iod, Trifluormethyl, Trifluormethoxy, Difluormethoxy, Trifluormethylsulfonyl, Alkylthio mit 1 bis 4 Kohlenstoffatomen, Nitro oder Cyano substituiert sein können,

$R^3$ -für eine Gruppe der Formel -CN oder -$COOR^5$ steht,
worin
$R^5$ -die gleiche Bedeutung hat wie $R^1$ und mit diesem gleich oder verschieden sein kann
und

$R^4$ -für Wasserstoff steht, oder
-für Phenyl steht, das bis zu 2-fach gleich oder verschieden durch Nitro, Cyano, Methyl, Methoxy, Fluor, Chlor, Brom, Iod, Trifluormethyl oder Trifluormethoxy substituiert sein kann,
sowie ihre pharmazeutisch unbedenklichen Salze.

Besonders bevorzugt seien Verbindungen der allgemeinen Formel (I) genannt, in welcher

$R^1$ -für einen geradkettigen, verzweigten oder cyclischen Kohlenwasserstoffrest mit bis zu 8 Kohlenstoffatomen steht, der durch ein Sauterstoffatome in der Kette unterbrochen sein kann und/oder der durch Fluor, Cyano, Acetoxy, Hydroxy, Phenyl, Phenoxy, α-, β-oder γ-Pyridyl oder durch eine Aminogruppe substituiert sein kann, wobei diese Aminogruppe zwei gleiche oder verschiedene Substituenten aus der Gruppe Alkyl mit 1 bis 2 Kohlenstoffatomen und Benzyl trägt,

$R^2$ -für Phenyl, Naphthyl, Thienyl, Pyridyl oder Benzoxadiazolyl steht, wobei die genannten Ringsystem durch 1 oder 2 gleiche oder verschiedene Substituenten aus der Gruppe Chlor, Trifluormethyl, Nitro oder Cyano substituiert sein können,

$R^3$ -für eine Gruppe der Formel -CN oder $COOR^5$ steht,
worin
$R^5$ -die gleiche Bedeutung hat wie $R^1$ und mit diesem gleich oder verschieden sein kann,
und

$R^4$ -für Wasserstoff steht, oder
-für Phenyl steht, das durch Nitro, Fluor, Chlor oder Trifluormethyl substiuiert sein kann,
sowie ihre pharmazeutisch unbedenklichen Salze.

Die erfindungsgemäßen Verbindungen der allgemeinen Formel (I) erhält man,
indem man
Ylidenverbindungen der allgmeinen Forme (II)

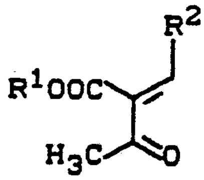

(II)

in welcher
$R^1$ und $R^2$ die oben angegebene Bedeutung haben,
in inerten Lösemitteln mit Cyanoethenen der allgemeinen Formel (III)

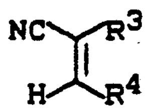

(III)

in welcher
$R^3$ und $R^4$ die oben angegebene Bedeutung haben,
mit Ammoniak oder einem geeigneten Ammoniumsalz umsetzt.

Verwendet man als Ausgangsstoffe 2-Trifluorbenzylidenacetessigsäure-ethylester, Benzylidenmalondinitril und Ammoniak so läßt sich das erfindungsgemäße Verfahren durch folgendes Schema verdeutlichen:

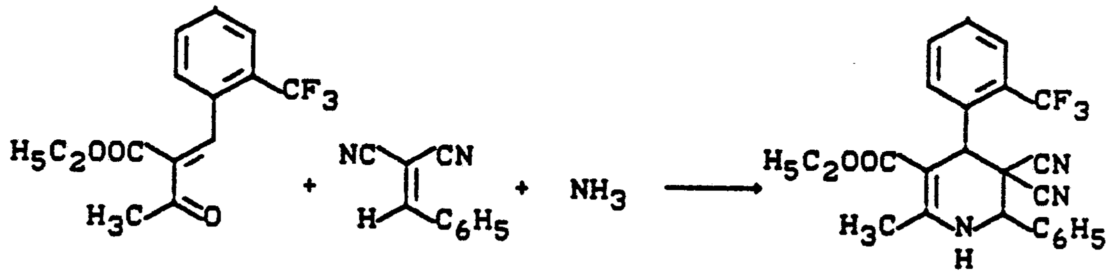

Als inerte Lösemittel eignen sich Wasser oder organische Lösemittel, die sich unter den Reaktionsbedingungen nicht verändern. Hierzu gehören bevorzugt Alkohle wie Methanol, Ethanol, Propanol oder Isopropanol, oder halogenkohlenwasserstoffe wie Methylenchlorid, Chloroform, Tetrachlorkohlenstoff, 1,2-Dichlorethan oder Dichlorethylen, oder Ether wie Diethylether, Dioxan oder Tetrahydrofuran, oder Kohlenwasserstoffe wie Benzol, Toluol oder Xylol, oder Amide wie Dimethylformamid oder Hexamethylenphosphorsäuretriamid, oder Ammoniak oder Eisessig. Ebenso ist es möglich, Gemische der genannten Lösemittel einzusetzen.

Die Reaktion wird im allgemeinen in einem Temperaturbereich von 0°C bis +200°C, bevorzugt von +20°C bis +150°C durchgeführt.

Die Reaktion kann bei Normaldurck, bei erhöhtem oder bei erniedrigtem Druck durchgeführt werden. Im allgemeinen arbeitet man bei Normaldruck.

Die Reaktion wird im allgemeinen so durchgeführt, daß man die Ylidenverbindung und das Cyanoethen in einem geeigneten Lösemittel mit konz. Ammoniaklösung, gegebenenfalls mit einem geeigneten Ammoniumsalz, mischt und gegebenfalls erwärmt. Die Aufarbeitung erfolgt im allgemeinen durch Extraktion, Chromatographie und/oder Kristallisation.

Geeignete Ammoniumsalze können Salze von Ammoniak mit anorganischen oder organischen Säuren sein. Es seien beispielhaft aufgeführt: Halogenide wie Chloride oder Bromide, Sulfate, Hydrogensulfate, Hydrogenphosphate, Carbonate, Hydrogencarbonate oder Acetate.

Besonders bevorzugt wird Ammoniumacetat eingesetzt. In diesen Fall wird besonders bevorzugt Essigsäure als Lösemittel verwendet.

Bei der Durchführung der Reaktion wird im allgemeinen die Ylidenverbindung in einer Menge von 1 bis 5 Mol, bevorzugt von 1 bis 2,5 Mol bezogen auf 1 Mol der Cyanoethenverbindung eingesetzt. Ammoniak bzw. ein geeignetes Ammoniumsalz wird im allgemeinen in einer Menge von 1 bis 10, bevorzugt von 1 bis 5 Mol, bezogen auf 1 Mol der Ylidenverbindung eingesetzt.

Die als Ausgangsstoffe eingesetzten Ylidenverbindungen sind bekannt oder können nach bekannten Methoden hergestellt werden [G. Jones "The Knoevenagel Condensation" in Organic Reaktions XV. 204 (1967)].

Die Cyanoethenverbindungen der allgemeinen Formel (II) sind bekannt oder können nach bekannten Methoden hergestellt werden [Beilstein's Handbuch der organischen Chemie 3 (1), 134:9, 893].

Die neuen Verbindungen haben ein breites und vielseitiges pharmakologisches Wirkungsspektrum.

Im einzelnen konnten im Tierexperiment folgende Hauptwirkungen nachgewiesen werden:

1.) Die Verbindungen bewirken bei parenteraler, oraler oder perlingualer Zugabe eine deutliche und langanhaltende Erweiterung der Koronargefäße. Diese Wirkung auf die Koronargefäße wird durch einen gleichzeitigen nitrit-ähnlichen herzentlastenden Effekt verstärkt. Sie beeinflussen bzw. verändern den Herzstoffwechsel im Sinne einer Energieersparnis.

2.) Die Erregbarkeit des Reizbildungs-und Erregungsleitungssystems innerhalb des Herzens wird herabgesetzt, so daß eine in therapeutischen Dosen nachweisbare Antiflimmerwirkung resultiert.

3.) Der Tonus der glatten Muskulatur der Gefäße wird unter der Wirkung der Verbindugen stark vermindert. Diese gefäßspasmolytische Wirkung kann im gesamten Gefäßsystem stattfinden, oder sich mehr oder weniger isoliert an beschriebenen Gefäßgebieten manifestieren.

4.) Die Verbindungen senken den Blutdruck von normotonen und hypertonen Tieren und können somit als antihypertensive Mittel verwendet werden.

5.) Der Verbindungen haben stark muskulärspasmolytische Wirkung, die an der glatten Muskulatur des Magen-Darm-Traktes, des Urogenitaltraktes und des Respirationssystems verdeutlicht werden.

Die erfindungsgemäßen Verbindungen eignen sich aufgrund dieser Eigenschaften besonders zur Prophylaxe und zur Therapie der akuten und chronischen ischämischen Herzkrankheiten im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B.:Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumsalfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriber Suspensionen können die Wirkstoffe außer den obengenannten Hilfstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erweisen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der

vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

3-Cyano-6-methyl-2,4-diphenyl-1,2,3,4-tetrahydropyridin-3,5-dicarbonsäure-diethylester

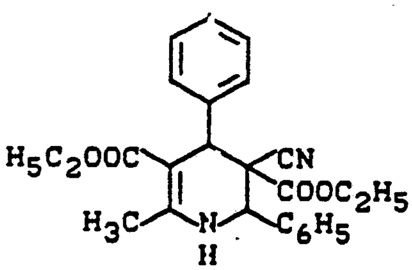

Einer Mischung aus 11,0 g (0,5 mol) Benzylidenacetessigsäureethylester, 10,0 g (0.05 mol) α-Cyanozimtsäureethylester, 50 ml Ethanol und 5,5 ml konz. Ammoniaklösung wird 1 d bei Raumtemperatur gerührt. Nach Eindampfen wird der Rückstand aus Isopropanol umkristallisiert.
Ausbeute: 10,5 g
Schmp.: 142°C

Beispiel 2

3-Cyano-6-methyl-4-(3-nitrophenyl)-1,2,3,4-tetrahydropyridin-3,5-dicarbonsäure-diethylester

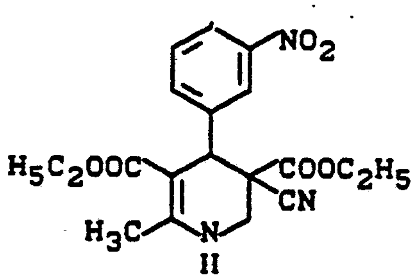

Eine Mischung aus 2,6 g (0.01 mol) 3-Nitrobenzylidenacetessigsäureethylester, 20 ml Essigsäure, 2,4 g (0,03 mol) Ammoniumacetat und 1,3 g (0,01 mol) α-Cyanoacrylsäureethylester wird 10 h bei 100°C gerührt. Nach Abkühlen wird auf eine Eis-Wasser-Mischung gegossen, die wäßrige Phase abgetrennt und der ölge Rückstand in Chloroform aufgenommen. Die organische Phase wird getrocknet und eingeengt. Der Rückstand wird durch Säulenchromatographie gereinigt.
Ausbeute: 0,8 g
Schmp.: 132°C

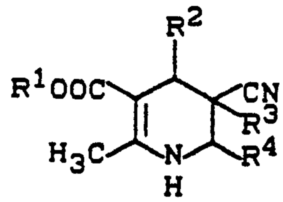

| Bsp.-Nr. | $R^1$ | $R^2$ | $R^3$ | $R^4$ | Fp. [°C] |
|---|---|---|---|---|---|
| 3 | $C_2H_5$ | 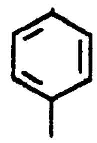 | $COOC_2H_5$ | 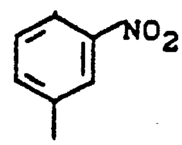 | 174 |
| 4 | $C_2H_5$ | 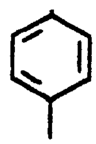 | CN | 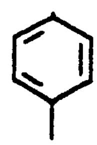 | 192 |
| 5 | $C_2H_5$ | 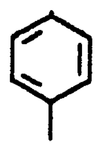 | $COOC_2H_5$ | 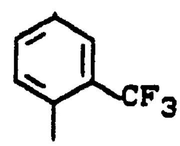 | 146 |
| 6 | $C_2H_5$ | 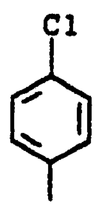 | $COOC_2H_5$ | 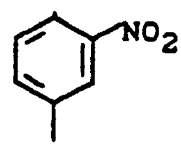 | 219 |
| 7 | $CH_3$ | 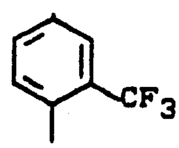 | $COOC_2H_5$ | H | 210 |
| 8 | $C_2H_5$ | 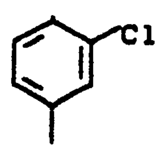 | $COOC_2H_5$ | H | 159 |

| Bsp.-Nr. | $R^1$ | $R^2$ | $R^3$ | $R^4$ | Fp. [°C] |
|---|---|---|---|---|---|
| 9 | $C_2H_5$ | 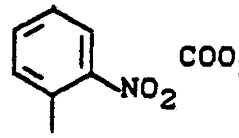 | $COO_2H_5$ | H | 159 |
| 10 | $C_2H_5$ | 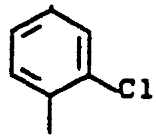 | $COOC_2H_5$ | H | 138 |
| 11 | $C_2H_5$ | 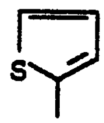 | $COOC_2H_5$ | H | 143 |
| 12 | $CH_3$ | 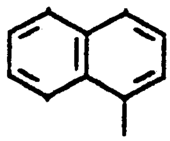 | $COOC_2H_5$ | H | 121 |
| 13 | $CH_3$ | 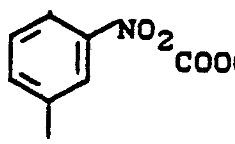 | $COOCH_2-CH(CH_3)_2$ | H | 158 |
| 14 | $C_2H_5$ | 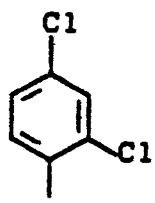 | $COOC_2H_5$ | H | 156 |
| 15 | $C_2H_5$ | 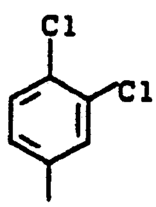 | $COOC_2H_65$ | H | 148 |

| Bsp.-Nr. | $R^1$ | $R^2$ | $R^3$ | $R^4$ | Fp. [° C] |
|---|---|---|---|---|---|
| 16 | $CH_3$ | 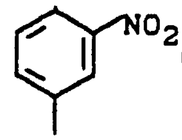 | $COOCH_3$ | H | 142 |
| 17 | $C_2H_5$ | 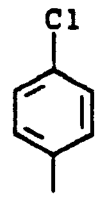 | $COOC_2H_5$ | H | 156 |
| 18 | $C_2H_5$ | 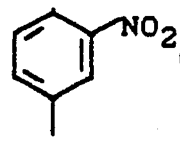 | $COOCH_3$ | H | 124 |
| 19 | $CH_3$ | 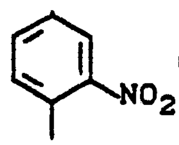 | $COOCH_3$ | H | 178 |
| 20 | $C_2H_5$ | 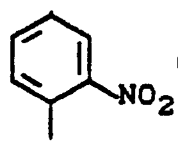 | $COOCH_3$ | H | 167 |

## Ansprüche

1. Tetrahydropyridine der allgemeinen Formel (I)

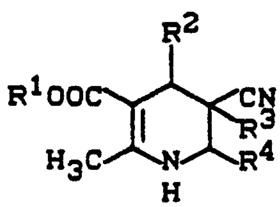

(I)

in welcher

$R^1$ -für einen geradkettigen, verzweigten oder cyclischen, gesättigten oder ungesättigten Kohlenwasserstoffrest mit bis zu 20 Kohlenstoffatomen steht, der durch ein Sauerstoffatom oder ein Schwefelatom in der Kette unterbrochen sein kann und/oder der substituiert sein kann durch Halogen, Cyano, Hydroxy, Acyloxy, Nitro, Nitrooxy oder durch eine gegebenenfalls durch Haolgen, Cyano, Dialkylamino mit jeweils 1 bis 2 Kohlenstoffatomen je Alkylgrupe, Alkoxy mit 1 bis 4 Kohlenstoffatomen, Alkyl mit 1 bis 4 Kohlenstoffatomen, Trifluormethyl oder Nitro substituierte Phenyl-, Phenoxy-, Phenylthio-oder Phenylsulfonylgruppe, oder durch eine α-, β-oder γ-Pyridylgruppe oder durch eine Aminogruppe, wobei diese Aminogruppe, 2 gleiche oder verschiedene Substituenten aus der Gruppe Alkyl mit bis zu 4 Kohlenstoffatomen, Alkoxyalkyl mit bis zu 4 Kohlenstoffatomen, Phenyl oder Aralkyl trägt oder wobei diese Substituenten gegebenenfalls mit dem Stickstaffatom einen fünf-bis siebengliedrigen Ring bilden, der als weiteres Heteroatom ein Sauerstoff-oder

Schwefelatom, oder eine N-Phenyl-oder N-Alkylgruppierung enthalten kann, wobei diese Alkylgruppe 1 bis 3 Kohlenstoffatome umfaßt,

$R^2$ -für Phenyl, Napthyl, Thienyl, Furyl, Pyrryl, Pyrazoyl, Imidazoly, Oxazolyl, Isoxazolyl, Thiazolyl, Pyridyl, Pyridazinyl, Pyrimidyl, Pyrazinyl, Indolyl, Benzimidazolyl, Benzoxazoyl, Benzisoxazolyl, Benzoxadiazolyl, Benzothiadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Trimethylen, Tetramethylen, Pentamethylen, Dioxymethylen, Dioxyethylen, Halogen, Trifluormethyl, Trifluormethoxy, Difluormethoxy, Tetrafluorethoxy, Nitro, Cyano oder $SO_m$-Alkyl, worin m eine Zahl von 0 bis 2 bedeutet und Alkyl vorzugsweise 1 bis 4 Kohlenstoffatome mit gegebenenfalls 1 bis 3 Fluoratomen enthält, substituiert sein können,

$R^3$ -für eine Gruppe der Formel -CN oder -$COOR^5$ steht,
worin
$R^5$ -die gleiche Bedeuting hat wie $R^1$ und mit diesem gleich oder verschieden sein kann
und

$R^4$ -für Wasserstoff steht, oder
-für Phenyl oder Naphthyl steht, wobei die genanntem Ringsysteme durch bis zu 2 gleiche oder verschiedene Substituenten der Reihe Nitro, Cyano, Alkyl mit 1 bis 4 Kohlenstoffatomen, Alkoxy mit 1 bis 4 Kohlenstoffatomen, Halogen, Trifluormethyl, Trifluormethoxy oder Trifluormethylthio,
sowie ihre pharmazeutisch unbedenklichen Säureadditionssalze.

2. Verbindungen der allgeinen Formel (I) gemäß Anspruch 1, in welcher

$R^1$ -für einen geradkettigen, verzweigten oder cyclischen, gesättigten oder ungesättigen Kohlenwasserstoffrest mit bis zu 14 Kohlenstoffatomen steht, der durch ein Sauerstoffatom in der Kette unterbrochen sein kann und/oder der substituiert sein kann durch Fluor, Chlor, Brom, Iod, Cyano, Hydroxy, Acetoxy, Nitrooxy oder durch eine gegebenenfalls durch Fluor, Chlor, Brom, Alkoxy mit 1 bis 4 Kohlenstoffatomen, Alkyl mit bis 1 bis 4 Kohlenstoffatomen oder Trifluormethyl substituierte Phenyl-oder Phenoxygruppe, oder durch eine α-, β-, γ-Pyridylgruppe oder durch eine Aminogruppe, wobei diese Aminogruppe 2 gleiche oder verschiedene Substituenten aus der Gruppe Alkyl mit 1 bis 4 Kohlenstoffatomen, Phenyl oder Benzyl trägt,

$R^2$ -für Phenyl, Naphthyl, Thienyl, Furyl, Pyridyl, Pyrimidyl, Chinolyl oder Benzoxadiazolyl steht, wobei die genannten Ringsysteme durch 1 bis 2 gleiche oder verschiedene Substituenten aus der Gruppe Phenyl, Alkyl mit 1 bis 4 Kohlenstoffatomen, Alkoxy mit 1 bis 2 Kohlenstoffatomen, Dioxymethylen, Fluor, Chlor, Brom, Iod, Trifluormethyl, Trifluormethoxy, Difluormethoxy, Trifluormethylsulfonyl, Alkylthio mit 1 bis Kohlenstoffatomen, Nitro oder Cyano substituiert sein können,

$R^3$ -für eine Gruppe der Formel - CN oder -$COOR^5$ steht,
worin
$R^5$ -die gleiche Bedeutung hat wie $R^1$ und mit diesem gleich oder verschieden sein kann
und

$R^4$ -für Wasserstoff steht, oder
-für Phenyl steht, das bis zu 2-fach gleich oder verschieden durch Nitro, Cyano, Methyl, Methoxy, Fluor, Chlor, Brom, Iod, Trifluormethyl oder Trifluormethoxy substituiert sein kann,
sowie ihre pharmazeutisch unbedenklichen Salze.

3. Verbindungen der allgemeinen Formel (I) gemäß Anspruch 1, in welcher

$R^1$ -für einen geradkettigen, verzweigten oder cyclischen Kohlenwasserstoffrest mit bis zu 8 Kohlenstoffatomen steht, der durch ein Sauerstoffatom in der Kette unterbrochen sein kann und/oder der durch Fluor, Cyano, Acetoxy, Hydroxy, Phenyl, Phenoxy, α-, β-oder γ-Pyridyl oder durch eine Aminogruppe substituiert sein kann, wobei diese Aminogruppe zwei gleiche oder verschiedene Substituenten aus der Gruppe Alkyl mit 1 bis 2 Kohlenstoffatomen und Benzyl trägt,

$R^2$ -für Phenyl, Napthyl, Thienyl, Pyridyl oder Benzoxadiazolyl steht, wobei die genannten Ringsysteme durch 1 oder 2 gleiche oder verschiedene Substituenten aus der Gruppe Chlor, Trifluormethyl, Nitro oder

Cyano substituiert sein können,

$R^3$ -für eine Gruppe der Formel -CN oder $COOR^5$ steht, worin
$R^5$ -die gleiche Bedeutung hat wie $R^1$ und mit diesem gleich oder verschieden sein kann, und

$R^4$ -für Wasserstoff steht, oder
-für Phenyl steht, das durch Nitro, Fluor, Chlor oder Trifluormethyl substituiert sein kann, sowie ihre pharmazeutisch unbedenklichen Salze.

4. Verfahren zur Herstellung von Verbindungen der allgemeinen Formel (I)

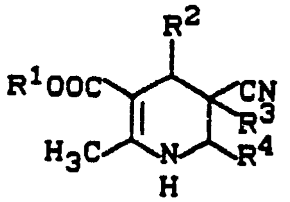

(I)

in welcher

$R^1$ -für einen geradkettigen, verzweigten oder cyclischen, gesättigten oder ungesättigten Kohlenwasserstoffrest mit bis zu 20 Kohlenstoffatomen steht, der durch ein Sauerstoffatom oder ein Schwefelatom in der Kette unterbrochen sein kann und/oder der substituiert sein kann durch Halogen, Cyano, Hydroxy, Acyloxy, Nitro, Nitrooxy oder durch eine gegebenenfalls durch Halogen, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substituierte Phenyl-, Phenoxy-, Pheylthio-oder Phenylsulfonylgruppe, oder durch eine α-, β-oder γ-Pyridylgruppe oder durch eine Aminogruppe, wobei diese Aminogruppe, 2 gleiche oder verschiedene Substituenten aus der Gruppe Alkyl mit bis zu 4 Kohlenstoffatomen, Alkoxyalkyl mit bis zu 4 Kohlenstoffatomen, Phenyl oder Aralkyl trägt oder wobei diese Substituenten gegebenenfalls mit dem Stickstoffatom einen fünf-bis siebengliedringen Ring bilden, der als weiteres Heteroatom ein Sauerstoff-oder Schwefelatom, oder eine N-Phenyl-oder N-Alkylgruppierung enthalten kann, wobei diese Alkylgruppe 1 bis 3 Kohlenstoffatome unfaßt,

$R^2$ -für Phenyl, Napthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzothiadiazolyl, Chinolyl, Isochinolyl, Chinazolyl oder Chinoxalyl steht, wobei die genannten Ringsysteme jeweils durch 1 oder 2 gleiche oder verschiedene Substituenten aus der Gruppe Phenyl, geradkettiges oder verzweigtes Alkyl mit 1 bis 8 Kohlenstoffatomen, Cycloalkyl mit 3 bis 7 Kohlenstoffatomen, Alkoxy mit 1 bis 4 Kohlenstoffatomen, Trimethylen, Tetramethylen, Pentamethylen, Dioxymethylen, Dioxyethylen, Halogen, Trifluormethyl, Trifluormethoxy, Difluormethoxy, Tetrafluorethoxy, Nitro, Cyano oder $SO_m$-Alkyl, worin m eine Zahl von 0 bis 2 bedeutet und Alkyl vorzugsweise 1 bis 4 Kohlenstoffatome mit gegebenenfalls 1 bis 3 Fluoratomen enthält, substituiert sein können,

$R^3$ -für eine Gruppe der Formel -CN oder $-COOR^5$ steht, worin
$R^5$ -die gleiche Bedeutung hat wie $R^1$ und mit diesem gleich oder verschieden sein kann und

$R^4$ -für Wasserstoff steht, oder
-für Phenyl oder Napthyl steht, wobei die genannten Ringsysteme durch bis zu 2 gleiche oder verschiedene Substituenten der Reihe Nitro, Cyano, Alkyl mit 1 bis 4 Kohlenstoffatomen, Alkoxy mit 1 bis 4 Kohlenstoffatomen, Halogen, Triflormethyl, Trifluormethoxy oder Trifluormethylthio substituiert sein können, und ihrer pharmazeutisch unbedenklichen Salze, dadurch gekennzeichnet, daß man Ylidenverbindungen der allgemeinen Formel (II)

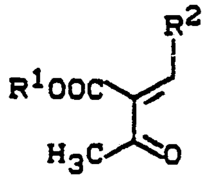

(II)

in welcher
$R^1$ und $R^2$ die oben angegebene Bedeutung haben,
in inerten Lösemitteln mit Cyanoethenen der allgemeinen Formel (III)

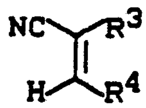

(III)

in welcher
$R^3$ und $R^4$ die oben angegebene Bedeutung haben,
mit Ammoniak oder einem geeigneten Ammoniumsalz umsetzt.

5. Verfahren gemäß Anspruch 4, dadurch gekennzeichnet, daß man die Umsetzung bei Temperaturen zweischen 0°C und 200°C durchführt.

6. Arzneimittel enthaltend wenigstens eine Verbindung der allgemeinen Formel (I) gemäß Anspruch 1.

7. Verfahren zur Herstellung von Arzneimitteln gemäß Anspruch 6, dadurch gekennzeichnet, daß man mindestens eine Verbindung der allgemeinen Formel (I) gemäß Anspruch 1 gegebenenfalls unter Verwendung von Hilfs-und Trägerstoffen in eine geeignete Applikationsform überführt.

8. Verwendung von Verbindungen der allgemeinen Formel (I) gemäß Anspruch 1 bei der Herstellung von Arzneimitteln.

9. Verwendung von Verbindungen der Allgemeinen Formel (I) gemäß Anspruch 1 bei der Hersellung von kreislaufbeeinflussenden Arzneimitteln.

10. Verwendung von Verbindungen der allgemeinen Formel (I) gemäß Anspruch 1 bei der Bekämpfung von Kreislauferkrankungen.

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

Nummer der Anmeldung

EP 87119218.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 110 073 (BAYER)<br>* Zusammenfassung; Ansprüche 1,9 *<br>-- | 1,6 | C 07 D 211/78<br>C 07 D 409/04<br>A 61 K 31/44 |
| A | DE - A1 - 3 414 801 (BAYER)<br>* Ansprüche 1,7 *<br>-- | 1,6 | |
| A | CHEMICAL ABSTRACTS, Band 79, Nr. 15, 15. Oktober 1973, Columbus, Ohio, USA<br>M.F. CHASLE-POMMERET et al. "Reactions of trivalent phosphorus compounds with positive halogen compounds"<br>Seite 418, Spalte 2, Zusammenfassung-Nr. 91 966q<br>& Tetrahedron, 29(10), 1419-27 (1973)<br>---- | 1 | |

RECHERCHIERTE SACHGEBIETE (Int. Cl.4)

C 07 D 211/00
C 07 D 409/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: -
Nicht recherchierte Patentansprüche: 10
Grund für die Beschränkung der Recherche:

(Verfahren zur therapeutischen Behandlung des menschlichen oder tierischen Körpers; Artikel 52(4) EPÜ)

| Recherchenort | Abschlußdatum der Recherche | Prüfer |
|---|---|---|
| WIEN | 08-04-1988 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82